# EUROPEAN PATENT SPECIFICATION

(11) **EP 4 319 828 B1**
(45) Date of publication and mention of the grant of the patent: **26.03.2025**
(21) Application number: 22720664.6
(22) Date of filing: 05.04.2022
(51) Int. Cl.: A61M 1/16, B01D 65/10, G01M 3/00, B01D 61/00

(54) **EVALUATING INTEGRITY OF A FORWARD OSMOSIS MEMBRANE DURING CONTINUOUS FLOW**
BEWERTUNG DER INTEGRITÄT EINER VORWÄRTSOSMOSEMEMBRAN WÄHREND EINES KONTINUIERLICHEN FLUSSES
ÉVALUATION DE L'INTÉGRITÉ D'UNE MEMBRANE D'OSMOSE DIRECTE PENDANT UN FLUX CONTINU

(30) Priority: 09.04.2021 US 202163172853 P; 12.08.2021 SE 2150992
(43) Date of publication of application: 14.02.2024
(73) Proprietor: Gambro Lundia AB, 226 43 Lund (SE)
(72) Inventor: VARTIA, Christian, 247 64 Veberöd (SE)
(74) Representative: Sweden SHS IP Office
(86) International application number: PCT/EP2022/058922
(87) International publication number: WO 2022/214446

(56) References cited:
- WO-A1-2015/124716
- WO-A1-2017/040696
- US-A1- 2012 199 526

## Description

### Technical field

The present disclosure relates to the field of dialysis and to integrity testing of forward osmosis membranes, and in particular to evaluating the integrity of a forward osmosis membrane arranged in a dialysis solution generation apparatus.

### Background

Dialysis is commonly used for treating patients suffering from renal failure. Several types of dialysis treatments exist such as hemodialysis (HD), peritoneal dialysis (PD) and continuous renal replacement therapy (CRRT). Typically, a dialysis fluid is used in the treatment and is delivered ready-made in bags or is produced at the point of use by mixing concentrate and water Document WO2017040696 deals with a water purification systems and methods having pressurized draw stream.

Forward osmosis (FO) has emerged as an option for producing dialysis fluid as it has potential for reducing water consumption. A FO-membrane is typically designed to be more or less exclusively selective towards water molecules, which enables the FO-membrane to separate water from all other contaminants. However, undetected integrity issues could allow transport of components other than water across the FO-membrane.

Thus, there is a need to detect such integrity issues to not compromise the produced dialysis fluid.

### Summary

A FO-membrane of the present disclosure is used to prepare dialysis fluid. The FO-membrane is in one embodiment more or less exclusively selective towards water molecules, which enables the FO-membrane to separate water from all other contaminants. An osmotic pressure difference between a feed fluid (for example water or effluent from the dialysis treatment) and a draw fluid (a dialysis concentrate) separated by the FO-membrane is used for extracting pure water from the feed fluid to the dialysis concentrate, thereby diluting the dialysis concentrate. The diluted dialysis concentrate is thereafter used for producing dialysis fluid. The control arrangements and methods of the present disclosure detect integrity issues with the FO-membrane, enabling the alteration of the composition of the produced dialysis fluid by the transport of components other than water across the FO-membrane to be prevented.

It is accordingly an objective of the disclosure to provide a simple and reliable method to evaluate the integrity of a forward osmosis membrane. It is a further objective to provide a method for evaluating the integrity of a forward osmosis membrane online or as dialysis fluid is being prepared.

These objectives and others are at least partly achieved by the method, control arrangement and dialysis solution generation apparatus according to the independent claims, and by the embodiments according to the dependent claims.

According to a first aspect, which may be combined with any other aspect and embodiment thereof, the disclosure relates to a method for evaluating integrity of a forward osmosis, FO, membrane of a FO-device in a dialysis solution generation apparatus. The FO-device is configured to be used in a FO session for diluting a dialysis concentrate in a process of producing a dialysis solution. The FO-membrane separates a first side from a second side of a FO-device. The method comprises passing electrolyte solution at the first side and passing low-electrolyte solution at the second side of the FO-membrane. The method further comprises measuring the conductivity of a solution generated from the second side and evaluating the integrity of the FO-membrane based on whether the measured conductivity meets conductivity criteria, wherein the conductivity criteria include or define the conductivity of a solution generated from the second side with an equivalent electrolyte solution and an equivalent low-electrolyte solution using a FO-membrane that is intact or has integrity.

The provided method evaluates the integrity of a FO-membrane in a simple and reliable way, by evaluating how well conductivity of a solution generated from the second side corresponds to an expected conductivity of a solution generated from the second side using the equivalent, hence same, solutions and a FO-membrane having integrity. The method may be performed with the same apparatus in which the FO-membrane is installed for use, whereby integrity of the FO-membrane may be tested at almost any time in the same apparatus, hence online. Also, the same fluids that will later be used for production may be used in the method.

According to some embodiments, evaluating the integrity comprises determining a lack of integrity of the FO-membrane, upon determining that a measured conductivity indicates a conductivity change from a conductivity of the low-electrolyte solution that is greater than a threshold. Hence, by using the conductivity of the low-electrolyte solution as a baseline, a robust integrity test may be made.

According to some embodiments, the threshold is based on a conductivity of the low-electrolyte solution and a predetermined dilution ratio of the low-electrolyte solution in the FO-device. Hence, the threshold is based on an anticipated solute diffusion over the FO-membrane.

According to some embodiments, the method comprises passing the electrolyte solution at the first side at a hydrostatic pressure that is lower than a hydrostatic pressure at the second side, thereby disabling convective transport from the first side to the second side, whereby a determined lack of integrity of the FO-membrane is indicative of a solute diffusive error of the FO-membrane. Thereby, a source of an integrity error may be further specified.

According to some embodiments, the method comprises passing the electrolyte solution at the first side at a hydrostatic pressure that is higher than the hydrostatic pressure at the second side, measuring the conductivity of the solution generated from the second side, and determining that the lack of integrity of the FO-membrane is indicative of a leak in the FO-membrane upon determining that a measured conductivity indicates a greater conductivity change than a conductivity change detected with the lower hydrostatic pressure at the first side. Thereby, one or more sources of an integrity error may be further specified.

According to some embodiments, the method comprises passing low-electrolyte solution at the second side at an osmotic pressure that is higher than the osmotic pressure at the first side, enabling diffusive water transport from the first side to the second side. Hence, the osmotic pressure difference between the sides may mimic the actual conditions during dialysis fluid production, allowing integrity testing under more realistic conditions.

According to some embodiments, the method comprises passing low-electrolyte solution at the second side at an osmotic pressure that is lower than the osmotic pressure at the first side, enabling diffusive water transport from the second side to the first side. Hence, a low-electrolyte solution such as water may be used, which is a solution that is cheaper than other low-electrolyte solutions such as glucose.

According to some embodiments, the low-electrolyte solution is a glucose solution. Hence, a readily available solution with a conductivity close to zero is used, whereby any electrolyte solution that mixes with the low-electrolyte solution is easily detected with conductivity sensing.

According to some embodiments, the low-electrolyte solution is water. Hence, a readily available low-cost liquid with low conductivity is used, whereby any electrolyte solution that mixes with the low-electrolyte solution is easily detected with conductivity sensing.

According to some embodiments, the electrolyte solution is effluent from a dialysis treatment. Hence, a readily available electrolyte solution may be used.

According to some embodiments, the electrolyte solution is a diluted electrolyte concentrate. Hence, a readily available electrolyte solution may be used.

According to some embodiments, wherein the low-electrolyte solution has a conductivity in a range from zero to 0.5 mS/cm, and in one embodiment below 0.1 mS/cm. Hence, any electrolyte solution that mixes with the low-electrolyte solution is easily detected with conductivity sensing.

According to a second aspect, which may be combined with any other aspect and embodiment thereof, the disclosure relates to a control arrangement for evaluating the integrity of a forward osmosis, FO, membrane of a FO-device in a dialysis solution generation apparatus. The FO-device is configured to be used in a FO session for diluting a dialysis concentrate in a process of producing a dialysis solution, wherein the FO-membrane separates a first side from a second side of the FO-device. The control arrangement comprises an effluent pump configured to provide a flow of electrolyte solution, a concentrate pump configured to provide a flow of low-electrolyte solution, and a conductivity sensor configured to sense a conductivity of a solution generated from the second side. The control arrangement is configured to: pass electrolyte solution at the first side, using the effluent pump, and to pass low-electrolyte solution at the second side, using the concentrate pump. The control arrangement is further configured to measure the conductivity, using the conductivity sensor, of a solution generated from the second side. The control arrangement is further configured to evaluate the integrity of the FO-membrane based on whether the measured conductivity meets conductivity criteria, wherein the conductivity criteria include or define the conductivity of a solution generated from the second side with an equivalent electrolyte solution and an equivalent low-electrolyte solution and using a FO-membrane that is intact or has integrity.

According to some embodiments, the control arrangement is configured to perform any embodiment described herein according to the first aspect.

According to a third aspect, which may be combined with any other aspect and embodiment thereof, the disclosure relates to a solution generation apparatus for generating dialysis solution. The apparatus comprises a forward osmosis device comprising a FO-membrane that separates a first side and a second side of the FO-device. The apparatus further comprises a control arrangement according to the second aspect.

According to a fourth aspect, the disclosure relates to a computer program comprising instructions to cause the control arrangement according to the second aspect to execute the method according to the first aspect.

According to a fifth aspect, the disclosure relates to a computer-readable medium having stored thereon the computer program of the fourth aspect.

### Brief description of the drawings

Fig. 1 is a schematic illustration of a FO-device, according to some embodiments of the disclosure.
Fig. 2 illustrates an example dialysis solution generation apparatus according to some embodiments of the disclosure.
Fig. 3 illustrates a method for evaluating the integrity of a FO-membrane of a FO-device according to some embodiments of the disclosure.
Fig. 4 is a diagram illustrating test results obtained from performing the method illustrated in Fig. 3.

### Detailed description

**In** the following description, methods for evaluating integrity of a forward osmosis (FO)-membrane will be described. The FO-membrane is used in a FO-device in a dialysis fluid generation apparatus for generating a dialysis solution that is thereafter used for producing a dialysis fluid. Membrane integrity may be defined as the quality or state of the complete membrane in perfect condition. Hence, a FO-membrane that has integrity is intact; it is not damaged or impaired in any way. The FO-membrane may experience a compromised integrity due to for example manufacturing errors or wear. The compromised integrity may cause integrity issues such as leakage (convective transport), or selectivity deterioration, which will cause an elevated rate of diffusive solute transport (solute flux) through the FO-membrane. Undetected integrity issues could alter the composition of produced dialysis fluid by allowing transport of components other than water across the FO-membrane. For example, a leakage may allow transport of microbials from the feed side (effluent or tap water) to the draw side (mixing side) and increase the risk for, e.g., peritonitis in case of PD. Further, a leakage may allow transport of solutes (electrolytes, glucose, urea etc.) from the feed side (effluent or tap water) to the draw side (mixing side) and thereby alter the composition of the produced dialysis fluid. Also, an elevated rate of diffusive electrolyte transport may be indicative of a risk that solutes (electrolytes, glucose, urea etc.) will diffuse across the FO-membrane in one or both directions at a rate that severely alters the composition of the produced dialysis fluid.

It has been found, as set forth in the present disclosure, that it is possible to detect such integrity issues after the FO-membrane is installed in the dialysis fluid generation apparatus, by using appropriate feed and draw solutions, and based on conductivity measurements of a generated solution. Depending on which solutions are used, the side that is normally referred to as draw side may instead be the feed side. Hence, hereinafter, the sides of the FO-device will be referred to as a first side and a second side, and the generated solution is the solution generated from the second side. By having a low electrolyte solution at the second side, small leakages or small elevated rates of diffusive solute transport into the second side may also be detected by the conductivity measurements of the generated solution, as it is predetermined what conductivity the generated solution should have using a FO-membrane having integrity at the same operating conditions. Convective fluid flow from first side to second side (feed side to draw side during production) is of concern since it poses a risk of carrying, e.g., patient effluent into the fluid production side and thereby alter the composition of the produced fluid. Convective fluid flow in the opposite direction is of less concern since it will not alter the composition of the produced fluid. Due to the possibility that a leak may act as a backflow valve and only allow leakage in one direction, it is desirable that a leak detection method tests for leakage in a direction of most interest (feed side to draw side). Therefore, the conductivity of the solution generated from the second side is here tested.

Generally, diffusive transport of water and solutes is driven by a solute concentration difference, i.e., a concentration difference between first side and second side. Convective flow (caused by a leak) is driven by a transmembrane pressure (TMP) difference, i.e., a pressure difference between first side and second side.

In some embodiments, the evaluation relies on existing technology and concentrates used for the production of dialysis fluid. For example, a conductivity sensor is already present for sensing conductivity of the solution generated from the second side during production of dialysis fluid, and that same sensor may be used for the present evaluation. The solution passed at the second side is for example a glucose solution or pure water that is already connected to the dialysis fluid generation apparatus. The solution at the first side is for example effluent from a dialysis treatment that may be readily available in an effluent container, or taken from the patient directly during an ordinary dialysis treatment. Alternatively, the solution at the first side is a diluted electrolyte solution, also referred to as a buffer solution. The diluted electrolyte solution may then be diluted to an osmolarity substantially below the osmolarity of the solution at the second side.

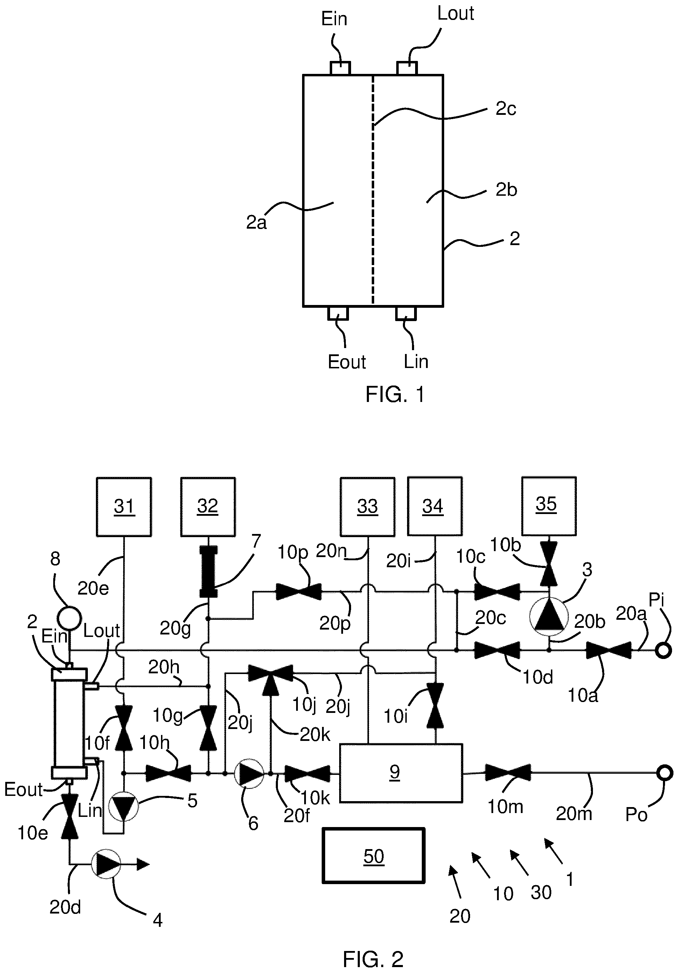

Embodiments of the disclosure will now be described with reference to Figs. 1 to 4. Fig. 1 is a schematic illustration of a FO-device 2 in isolation according to some embodiments. The FO-device 2 comprises a first side 2a, for example, a feed side, and a second side 2b, for example, a draw side, that is separated by a FO-membrane 2c. A side may also be referred to herein as a compartment or chamber. The FO-device 2 typically includes a cartridge that encloses the first side 2a, second side 2b and FO-membrane 2c. The geometry of the FO-membrane 2c may be a flat-sheet, or a tubular or hollow fiber. The FO-membrane 2c is a water permeable membrane. The FO-membrane 2c is designed to be more or less exclusively selective towards permeating water molecules, which enables the FO-membrane 2c to separate water from all other contaminants. The FO-membrane 2c typically has a pore-size in the nanometer (nm) range, for example, from 0.5 to 5 nm or less depending on the solutes that are intended to be blocked. During use, the FO-membrane 2c separates a solution at the feed side (typically a feed solution) and a solution at the second side (typically a draw solution). The fluids at these sides typically flow in counter-current flow but may alternatively flow in co-current flows. The flows are continuous flows in one embodiment, hence, the flows are flowing uninterrupted. The first side 2a has an inlet port *Eᵢₙ* where a solution passes into the first side 2a, and an outlet port *Eₒᵤₜ* wherefrom the solution passes out from the first side 2a. The second side 2b has an inlet port *Lᵢₙ* where another solution passes into the second side 2b, and an outlet port *Lₒᵤₜ* wherefrom the other solution passes out from the second side 2b. One of the solutions becomes dewatered, and the other one becomes diluted, depending on the osmotic pressure difference between the solutions. Suitable FO-devices for FO-device 2 may be provided by, e.g., Aquaporin^{™}, AsahiKASEI^{™}, Berghof^{™}, CSM^{™}, FTSH₂O^{™}, Koch Membrane Systems^{™}, Porifera^{™}, Toyobo^{™} and Toray^{™}.

To generate the dialysis solution, water is extracted from a feed solution at the first side 2a to a draw solution at the second side 2b by means of an osmotic pressure difference. The feed solution is for example water or effluent. In some embodiments, the effluent has an osmotic pressure of about 8 bar (116 psig). The draw solution is for example a dialysis concentrate, and the extracted water dilutes the dialysis concentrate into a dialysis solution, which may also be referred to as a "diluted dialysis concentrate", an "intermediate dialysis solution"," or simply "dialysis solution". In this disclosure, the dialysis concentrate may be referred to as an electrolyte solution, and a diluted dialysis concentrate may be referred to as a diluted electrolyte solution. The dialysis concentrate is for example a concentrate including at least one of, e.g., a plurality of, NaCl, KCl, CaCl2, MgCl2, HAc, glucose, lactate and bicarbonate. For example, the dialysis concentrate may comprise NaCl, CaCl2, MgCl2 and Na-lactate.

In some embodiments, the dialysis concentrate has an osmotic pressure of about 70 bar (1015 psig). The dialysis fluid may be used for PD, HD, CRRT or any other dialysis treatment using dialysis fluid as a treatment fluid or replacement fluid (e.g., for diluting blood post-filtering).

In this disclosure, an electrolyte solution is a solution with a concentration of electrolytes high enough to yield a significant conductivity response on the second side if the FO-membrane's integrity is compromised. For example, the concentration of electrolytes is 300 to 500 milli Molar (mM), which is at least 20 times greater that the concentration of electrolytes in a low-electrolyte solution. Such electrolyte solution is for example effluent from a dialysis treatment, or a diluted electrolyte solution. Effluent may here include patient effluent in PD and/or spent dialysate in HD. A low-electrolyte solution is a solution with a very low concentration of electrolytes. In some embodiments, the low-electrolyte solution has a conductivity in a range from zero to 0.5 mS/cm, and in one embodiment below 0.1 mS/cm. Hence, the conductivity of the low-electrolyte solution is very low. The low-electrolyte solution is for example a glucose solution or pure water.

The pure water typically has a quality as water for injection (WFI) or water for dialysis (WFD). WFI has a Total Organic Carbon (TOC) of maximum 500 ppg, a conductivity at 25°C of less than 1.3 µS/cm, and bacterial endotoxins at less than 0.25 EU/ml. WFD has a Colony Forming Unit (CFU) of less than 100 CFU/ml and an Endotoxin Unit of less than 0.25 EU/mL. See e.g. ISO 26722:2009, ISO 22519:2019.

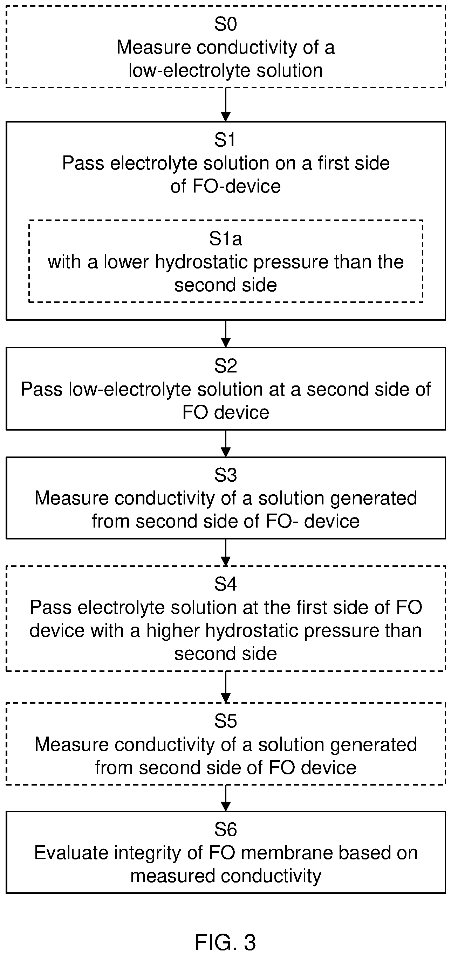

Fig. 2 illustrates a dialysis fluid generation apparatus 1 (hereinafter "apparatus 1") according to some embodiments of the disclosure. The apparatus 1 comprises a FO-device 2 as explained with reference to Fig. 1. The apparatus 1 also comprises a flow path 20 comprising a plurality of fluid lines 20a-20n, hereinafter referred to as "lines". The apparatus 1 further comprises a control arrangement 30. The control arrangement 30 comprises an effluent pump 3, a drain pump 4, a concentrate pump 5 and a diluted electrolyte pump 6. Any of the pumps herein may for example be a volumetric pump (such as a piston pump) or a non-volumetric pump (for example a gear pump) with flow rate feedback from a flow sensor (not shown). The effluent pump 3 is configured, depending on a valve state, to pump effluent from a patient to an effluent container 35. The effluent pump 3, depending on the valve state, is configured to provide a flow of effluent from the effluent container 35 or inlet connector *Pᵢ* to the first side 2a and from the first side 2a to a drain (not shown). The concentrate pump 5 is configured, depending on a valve state, to provide a flow of a solution from any of electrolyte solution container 31, pure water container 33 or fluid container 34 to the second side 2b. The electrolyte solution container 31 comprises an electrolyte solution. The pure water container 33 comprises pure water. The fluid container 34 comprises a low-electrolyte solution, for example, glucose. The control arrangement 30 also comprises a conductivity sensor 7 configured to sense a conductivity of a solution generated from the second side 2b. The conductivity sensor 7 is arranged to sense conductivity in the range of 0.01 to 40 mS/cm in one embodiment. The conductivity sensor may also include a temperature sensor (not shown) to compensate sensed conductivity values. The diluted electrolyte pump 6 is configured, depending on a valve state, to provide a flow of fluid in a main line 20f. The diluted electrolyte pump 6 is for example configured to provide a flow of fluid to the conductivity sensor 7 via line 20g from pure water container 33 or fluid container 34. This is performed so as to be able to measure the baseline conductivity of the low-electrolyte solution. The control arrangement 30 also comprises a valve arrangement 10, comprising a plurality of valves 10a-10m. Generally, a valve connected to a line may be configured as open, wherein a fluid flow in the line is allowed, and closed, wherein a fluid flow in the line is stopped. A valve may for example be an on/off valve, wherein the on state defines a state when fluid flow in the line is allowed, and the off state is a state wherein the fluid flow in the line is stopped. The control arrangement 30 further comprises a control unit 50 comprising at least one memory and at least one processor. The control arrangement 30 is configured to control the pumps 3-6 and the valves 10a-10m of the valve arrangement 10 to perform a plurality of different processes, such as to provide dialysis fluid, perform a cleaning process or a priming process. The control arrangement 30 is also configured to receive measurements of conductivity from the conductivity sensor 7. The control arrangement 30 is further configured to receive measurements of pressure from the pressure sensor 8. Specifically, the control arrangement 30 is configured to evaluate the integrity of the FO-membrane 2c in the apparatus 1, according to a method illustrated in Fig. 3. For that purpose, the at least one memory comprises instructions for evaluating integrity of the FO-membrane 2c. When the instructions are executed by the at least one processor, the control arrangement 30 performs the method for evaluating integrity of the FO-membrane 2c that will be explained in the following. The method may be performed by the control arrangement 30 and stored as a computer program including computer instructions on the at least one memory.

However, first the apparatus 1 in Fig. 2 will be described in more detail. In Fig. 2, a first effluent inlet line 20a is arranged between an inlet connector *Pᵢ* and an inlet port *Eᵢₙ* of the first side 2a, to connect the inlet connector *Pᵢ* and the inlet port *Eᵢₙ.* The inlet connector *Pᵢ* is for example connectable to a catheter of a PD patient, or to an effluent line of a HD or CRRT apparatus. A first effluent inlet valve 10a is connected to the first effluent inlet line 20a. A second effluent inlet line 20b is arranged between the first effluent inlet line 20a and an effluent container 35, to connect the first effluent inlet line 20a and the effluent container 35. An effluent pump 3 is arranged to provide a flow of effluent in the second effluent inlet line 20b. A second effluent inlet valve 10b is connected to the second effluent inlet line 20b. A third effluent inlet line 20c is arranged between the first effluent inlet line 20a and the second effluent inlet line 20b, to connect the first effluent inlet line 20a and the second effluent inlet line 20b. A third effluent inlet valve 10c is connected to the third effluent inlet line 20c. A fourth effluent inlet valve 10d is connected to the first effluent inlet line 20a between the connection of the second effluent inlet line 20b and the third effluent inlet line 20c. The effluent may be collected in the effluent container 35 by pumping effluent to the container 35 with the effluent pump 3, opening first effluent inlet valve 10a and second effluent inlet valve 10b, and closing third effluent inlet valve 10c and fourth effluent inlet valve 10d. Effluent can thereafter be pumped with effluent pump 3 from effluent container 35 to first side 2a by opening second effluent inlet valve 10b and fourth effluent inlet valve 10d, and closing first effluent inlet valve 10a and third effluent inlet valve 10c. Alternatively, effluent may be pumped directly to first side 2a by pumping effluent with effluent pump 3 from inlet connector *Pᵢ* through first effluent inlet line 20a, second effluent inlet line 20b and third effluent inlet line 20c, and opening first effluent inlet valve 10a and third effluent inlet valve 10c, and closing second effluent inlet valve 10b and fourth effluent inlet valve 10d. A pressure sensor 8 is arranged to sense the pressure in the first effluent inlet line 20a. The sensed pressure from the pressure sensor 8 is representative of the pressure at the first side 2a.

An effluent outlet line 20d is arranged between an outlet connector *Eₒᵤₜ* of the first side 2a and a drain, and connects the outlet connector *Eₒᵤₜ* of the first side 2a to drain. An effluent outlet valve 10e is connected to the effluent outlet line 20d. The drain pump 4 is arranged to provide a flow in the effluent outlet line 20d, and to control the hydrostatic pressure on the first side 2a. Effluent may be pumped from the outlet connector *Eₒᵤₜ* to the drain by opening effluent outlet valve 10e and pumping with drain pump 4. The effluent pump 3 and the drain pump 4 may thus pump jointly to provide a desired flow rate of effluent at the first side 2a, at a desired hydrostatic pressure. The pressure at the first side 2a is measured with the pressure sensor 8, and the speed of the drain pump 4 is controlled to achieve the desired hydrostatic pressure at the first side 2a, based on the pressure measured with the pressure sensor 8. The pressure at the second side 2b is assumed to be constant and close to atmospheric pressure, for example, 1013 hPa. In some embodiments, the pressure at the second side 2b is measured with another pressure sensor (not shown). Alternatively, only the drain pump 4 is used to provide the flow of effluent at the first side 2a. The second effluent inlet valve 10b, the third effluent inlet valve 10c and the effluent outlet valve 10e are then open, the effluent pump 3 is stopped, and the fourth effluent inlet valve 10d is closed.

Further, an electrolyte solution line 20e is arranged between the electrolyte solution container 31 and the inlet port *Lᵢₙ* of the second side 2b to connect the electrolyte solution container 31 and the inlet port *Lᵢₙ* of the second side 2b. An electrolyte solution valve 10f is connected to the electrolyte solution line 20e. Also, a concentrate pump 5 is arranged to provide a flow in the electrolyte solution line 20e. A main line 20f is arranged between the electrolyte solution line 20e and a mixing unit 9, and connects the electrolyte solution line 20e and the mixing unit 9. The mixing unit 9 includes fluid mixing functionality such as a main pump controlling a resulting flow rate in line 20m downstream mixing unit 9, a low-electrolyte solution pump providing a flow of low-electrolyte solution, a conductivity sensor, a heater and a mixing chamber (these features not explicitly shown). The main line 20f is connected to the electrolyte solution line 20e between the electrolyte solution valve 10f and the concentrate pump 5. A diluted electrolyte container line 20g is arranged between a diluted electrolyte container 32 and the main line 20f, to connect the diluted electrolyte container 32 and the main line 20f. The diluted electrolyte container 32 is used to accumulate electrolyte fluid after it has been diluted in a FO-session. A conductivity sensor 7 is connected to the diluted electrolyte container line 20g, in order to sense a conductivity of a fluid in the diluted electrolyte container line 20g. A diluted electrolyte container valve 10g is connected to the diluted electrolyte container line 20g. A first connecting line 20h is arranged between an outlet port *Lₒᵤₜ* of the second side 2b and the diluted electrolyte container line 20g, to connect the outlet port *Lₒᵤₜ* of the second side 2b and the diluted electrolyte container line 20g. A first main valve 10h is connected to the main line 20f, between a connecting point of the main line 20f and the electrolyte solution line 20e, and a connecting point of the diluted electrolyte container line 20g and the main line 20f. Electrolyte solution may be pumped from the electrolyte solution container 31 to the diluted electrolyte container 32 via the second side 2b, by opening electrolyte solution valve 10f, pumping with concentrate pump 5 and closing diluted electrolyte container valve 10g and first main valve 10h. Simultaneously, effluent may be passed, hence pumped, at the first side 2a. Pure water will then be extracted from the effluent at the first side 2a to the electrolyte solution at the second side 2b, by osmotic pressure. Thus, the electrolyte solution will become diluted to form an intermediate dialysis solution and is collected in diluted electrolyte container 32. This procedure may be referred to as a FO-session. Hence, the FO-device 2 is configured to be used in a FO-session for diluting a dialysis concentrate (the electrolyte solution) in a process of producing a dialysis solution.

A third connecting line 20p is arranged between the diluted electrolyte container line 20g and the third effluent inlet fluid 20c and connects the diluted electrolyte container line 20g and the third effluent inlet fluid line 20c. The third connecting line 20p is connected to the diluted electrolyte container line 20g between the conductivity sensor 7 and the diluted electrolyte container valve 10g. The third connecting line 20p is connected to the third effluent inlet fluid line 20c between the third effluent inlet valve 10c and its connection to the first effluent inlet line 20a. A valve 10p is connected to the third connecting line 20p. A fluid line 20i is arranged between the fluid container 34 and the mixing unit 9, to connect the fluid container 34 and the mixing unit 9. A solution valve 10i is connected to the fluid line 20i. A second connecting line 20j is arranged between the fluid line 20i and the main line 20f, to connect the fluid line 20i and the main line 20f. A three way-valve 10j is connected to the second connecting line 20j. A middle line 20k is arranged between the three-way valve 10j and the main fluid line 20f and connects the three-way valve 10j and the main fluid line 20f. A second main valve 10k is connected to the main line 20f, between the diluted electrolyte pump 6 and the mixing unit 9. A water line 20n is arranged between the pure water container 33 and the mixing unit 9, to connect the pure water container 33 and the mixing unit 9. An outlet line 20m is arranged between the mixing unit 9 and an outlet connector *Pₒ,* to connect the mixing unit 9 and the outlet connector *Pₒ.* The outlet connector *Pₒ* may for example be connected to a catheter of a PD patient, or to a dialysis fluid line of a HD or CRRT apparatus. An outlet valve 10m is arranged to the outlet line 20m.

For mixing a dialysis fluid, the diluted electrolyte solution in diluted electrolyte container 32 is pumped to mixing unit 9 by pumping with diluted electrolyte pump 6, opening diluted electrolyte container valve 10g, second main valve 10k and outlet valve 10m. At the same time, low-electrolyte solution such as glucose is passed to the mixing unit 9 by opening solution valve 10i, and pumping with a low-electrolyte solution pump (not shown). Pure water flows to the mixing unit 9 via water line 20n. The main pump (not shown) provides a desired flow rate of resulting dialysis fluid in the line 20m downstream mixing unit 9. A conductivity sensor (not shown) of the mixing unit 9 measures the conductivity of the resulting dialysis fluid from the mixing unit 9. The diluted electrolyte pump 6 and the low-electrolyte solution pump are controlled to certain speeds to achieve a desired predetermined concentration of the resulting dialysis fluid, which is based on the conductivity of the produced fluid, the conductivity of the diluted electrolyte solution and flow rate of the produced fluid. **In** the mixing unit 9, the diluted electrolyte solution, the low-electrolyte solution and the pure water are mixed in a mixing chamber to form a dialysis fluid, and may optionally be heated. Thereafter, the dialysis fluid is delivered at the outlet connector *Pₒ* via outlet line 20m to a desired destination (e.g., a storage container or a dialysis machine).

A method for evaluating integrity of a FO-membrane will now be explained with reference to the flow chart in Fig. 3. The method is for example implemented by the control unit 50 in Fig. 2. The FO-membrane is for example the FO-membrane 2c of the FO-device 2 in the apparatus 1 in Fig. 2. The method may be performed before a treatment starts, after a treatment has stopped, or both. In some embodiments, the method comprises measuring S0 conductivity of the low-electrolyte solution. The measuring S0 may comprise measuring the conductivity using the conductivity sensor 7. The measuring S0 will then include pumping a sample of the low-electrolyte solution to the conductivity sensor 7. For example, in the case where the low-electrolyte solution is a glucose solution, the method may comprise opening three-way valve 10j downwards towards main line 20f, opening diluted electrolyte container valve 10g, and pumping glucose solution from the fluid container 34 to the conductivity sensor 7 with the diluted electrolyte pump 6 in reverse direction. Alternatively, the conductivity of the low-electrolyte solution is previously known, obtained, measured or estimated. For example, in case the low-electrolyte solution is pure water, the conductivity is assumed to be zero. This conductivity may be used as a reference (baseline) for the conductivity of a solution generated from the second side 2b.

The method further comprises passing S1 electrolyte solution at the first side 2a. In other words, the method comprises providing a flow of electrolyte solution at the first side 2a. Hence, the electrolyte solution is provided at the inlet port *Eᵢₙ* at a certain flow rate, flows from the inlet port *Eᵢₙ* through the first side 2a for osmotic exchange through the FO-membrane 2c, to the outlet port *Eₒᵤₜ,* where the solution leaves the FO-device 2. The electrolyte solution is for example effluent or a diluted electrolyte concentrate. In the case where the electrolyte solution is effluent, the passing S1 includes pumping effluent from the effluent container 35, patient or other source fluidly connected to inlet connector Pi, and further to the inlet port *Eᵢₙ* of the first side 2a, using the effluent pump 3 and/or the drain pump 4 and opening/closing appropriate valves. In the case where the electrolyte solution is diluted electrolyte concentrate, the pumping comprises S1 pumping previously prepared diluted electrolyte concentrate from the diluted electrolyte container 32 to the effluent container 35. The pumping is then performed using effluent pump 3, pumping the diluted electrolyte concentrate through lines 20p, 20c, 20a, 20b, opening valves 10p, 10d, 10b and closing valves 10c, 10a. The effluent container 35 is then either empty, or if not, the effluent container 35 is next emptied to drain. The passing S1 further comprises pumping the diluted electrolyte concentrate or effluent from the effluent container 35 to the inlet port *Eᵢₙ* of the first side 2a, using the effluent pump 3 and/or the drain pump 4, opening valves 10b, 10d and closing valves 10a, 10c, 10p. The passing S1 further includes pumping the electrolyte solution into the inlet port *Eᵢₙ* of the first side 2a, through the first side 2a and out from the outlet port *Eₒᵤₜ* of the first side 2a to a drain (not shown) and opening valve 10e. The hydrostatic pressure at both sides 2a, 2b may be close to atmospheric pressure.

In some embodiments, the method comprises passing S1 the electrolyte solution at the first side 2a at a hydrostatic pressure which is lower than the hydrostatic pressure at the second side 2b. Thereby, convective transport from the first side 2a to the second side 2b is disabled, enabling a more precise evaluation of the source to the integrity error to be made. In other words, the present embodiments comprise configuring the hydrostatic pressure P1 at the first side 2a such that it is lower than the hydrostatic pressure P2 at the second side 2b. The configuring is for example performed by measuring the hydrostatic pressure P1 at the first side 2a with the pressure sensor 8 and controlling a speed of the drain pump 4 based on the sensed pressure such that the hydrostatic pressure P1 is equal to the lower hydrostatic pressure. The hydrostatic pressure at the second side 2b is typically constant, as the outlet port *Lₒᵤₜ* is open to atmospheric pressure via the diluted electrolyte container 32. Hence, by changing the hydrostatic pressure P1 at the first side 2a, the transmembrane pressure (TMP) between the first side 2a and the second side 2b can be changed to a desired TMP.

The method further comprises passing S2 low-electrolyte solution at the second side 2b. The passing S2 of low-electrolyte solution is performed while passing S1 electrolyte solution at the first side 2a. **In** other words, the method comprises providing a flow of low-electrolyte solution at the second side 2b. Hence, the low-electrolyte solution is provided at the inlet port Lin at a certain flow rate, and flows from the inlet port *Lᵢₙ* through the second side 2b for osmotic exchange through the FO-membrane 2c, to the outlet port *Lₒᵤₜ,* where the solution leaves the FO-device 2. The low-electrolyte solution is for example glucose solution or pure water. **In** the case of glucose solution, the passing S2 comprises pumping glucose solution from the fluid container 34 to the inlet port *Lᵢₙ* of the second side 2b, using the concentrate pump 5 and opening valves 10j, 10h and closing valves 10i, 10k, 10g, 10f. In the case of pure water, the passing S2 comprises pumping pure water from the pure water container 33 to the inlet port *Lᵢₙ* of the second side 2b, using the concentrate pump 5 and optionally diluted electrolyte pump 6, opening valves 10k, 10h and closing valves 10i, 10m, 10j (closed to line 20j), 10g, 10f. The pure water is first pumped to mixing unit 9, wherefrom it is further pumped to the inlet port *Lᵢₙ.* The passing S2 further includes pumping the low-electrolyte solution into the inlet port *Lᵢₙ* of the second side 2b, through the second side 2b and out from the outlet port *Eₒᵤₜ* of the second side 2b. The low-electrolyte solution is thereafter pumped to the diluted electrolyte container 32, while the conductivity is sensed with conductivity sensor 7. The first step and the second step may be performed continuously and simultaneously during the testing, hence, the passing includes providing simultaneous continuous flows at the first side 2a and the second side 2b. The passing S1, S2 is performed to provide a certain flow rate of the electrolyte solution when it flows to the first side 2a, which together with a certain flow rate of the low-electrolyte solution to the second side 2b, results in a predetermined conductivity of the generated fluid from the second side 2b, if the FO-membrane is intact or has integrity.

Depending on an osmotic pressure difference between the electrolyte solution at the first side 2a and the low-electrolyte solution at the second side 2b, one of the solutions becomes dewatered and the other one becomes diluted. The electrolyte solution typically has an osmotic pressure around 8 bar (116 psig). In the case that the low-electrolyte solution is a glucose solution with sufficient concentration of glucose, the osmotic pressure at the second side 2b will be higher than at the first side 2a. A glucose solution with 6% glucose (remaining 94% typically pure water) has an osmotic pressure about 8 bar (116 psig). The glucose solution may therefore include more than 6% glucose, for example at least 10%, at least 20%, up to 50%. Hence, the glucose solution may include from 10% to 50% glucose to have a sufficient concentration of glucose. For a FO-membrane 2c having integrity, water will then diffuse from the electrolyte solution at the first side 2a to the glucose solution at the second side 2b and hence dilute the glucose solution. Hence, in some embodiments, the method comprises passing S2 low-electrolyte solution at the second side 2b at an osmotic pressure that is higher than the osmotic pressure at the first side 2a enabling diffusive water transport from the first side 2a to the second side 2b. This will mimic the real operation of a FO water extraction session with patient effluent or water on the first side 2a and dialysis concentrate on the draw side 2b. In the case where the low-electrolyte solution is pure water, the osmotic pressure at the second side 2b will be lower than at the first side 2a. The osmotic pressure of pure water is typically zero, or close to zero. For a FO-membrane 2c having integrity, water will then diffuse from the pure water at the second side 2b to the electrolyte solution at the first side 2a and hence dilute the electrolyte solution. Hence, in some embodiments, the method comprises passing S2 low-electrolyte solution at the second side at an osmotic pressure that is lower than the osmotic pressure at the first side 2a, enabling diffusive water transport from the second side 2b to the first side 2a. In the case of an integrity issue, such as leakage or abnormal diffusion, electrolytes will be transported to the second side 2b and the conductivity of the solution generated from the second side 2b will increase. For example, in the case of a diffusive error of the FO-membrane 2c, electrolytes may diffuse from the first side 2a to the second side 2b, whereby the conductivity of the solution at the second side 2b will increase. In the case of a leak in the FO-membrane 2c, electrolytes may leak from the first side 2a to the second side 2b, whereby the conductivity of the solution at the second side 2b will increase. Hence, the integrity of the FO-membrane 2c is evaluated by measuring conductivity of the low-electrolyte solution outputted from the second side 2b. The method accordingly comprises measuring conductivity S3 of a solution generated from the second side 2b. Measuring conductivity S3 may include monitoring the conductivity for a time period until the conductivity has stabilized. The measuring of the conductivity S3 is typically performed using the conductivity sensor 7. The method accordingly comprises pumping the solution generated from the second side 2b to the conductivity sensor 7, measuring the conductivity, and further pumping the solution to diluted electrolyte container 32.

The method further comprises evaluating integrity S6 of the FO-membrane 2c based on whether the measured conductivity meets one or more conductivity criteria, wherein the conductivity criteria include or define the conductivity of a solution generated from the second side with an equivalent electrolyte solution and an equivalent low-electrolyte solution using an intact FO-membrane having integrity. Hence, the method includes evaluating whether the FO-membrane 2c has integrity or not, based on an expected resulting conductivity of a solution generated from the second side 2a with a same kind of FO-membrane, having integrity, at the same operating point and with the same, equivalent, fluids. The conductivity criteria may include one or more thresholds for the conductivity of the fluid generated from the second side 2b. The one or more thresholds may be based on previous experiments using FO-membranes of the same type having integrity with the same solutions used, wherein acceptable thresholds for the conductivity are determined based on knowledge of anticipated and acceptable electrolyte diffusion over the FO-membrane 2c. Alternatively or in combination, the one or more thresholds may be based on the conductivity of the low-electrolyte solution. For example, the one or more thresholds may define an acceptable change, e.g., increase, from the conductivity of the low-electrolyte solution. Upon the change in conductivity being greater than a stored threshold change, a FO-membrane lacking integrity can be determined. Hence, in some embodiments, the evaluating integrity S6 comprises determining a lack of integrity of the FO-membrane 2c, upon the measured conductivity indicating a conductivity change from the conductivity of the low-electrolyte solution that is greater than a threshold. Hence, in such embodiments, the evaluating integrity S6 may include calculating the conductivity change from the conductivity of the low-electrolyte solution to the measured conductivity of the solution generated from the second side 2b and comparing the conductivity change to the threshold. The conductivity threshold for the generated solution at the second side 2b may also be given by predefined tabulated conductivity values, stored in memory of control unit 50, for a certain type and conductivity of the low-electrolyte solution.

The anticipated and acceptable electrolyte diffusion may be expressed as a predetermined dilution ratio of the low-electrolyte solution at the second side 2b. Hence, in some embodiments, the threshold is based on a conductivity of the low-electrolyte solution and a predetermined dilution ratio of the low-electrolyte solution in the FO-device 2. The dilution ratio is dependent on the solutions used, the flow rates and characteristics of the FO-membrane 2c. The dilution ratio may be defined as the volume/flow rate of low-electrolyte solution to the second side 2b, compared to the volume/flow rate of generated solution from the second side 2b. The dilution ratio may be assessed based on the current operating point (e.g., flow rates to the sides 2a, 2b) of the FO-device 2 and assumptions/knowledge about the electrolyte solution composition and of the low-electrolyte solution composition (e.g., their osmotic pressures). The actual conductivity change, e.g., increase or increment, can then be compared to this threshold and conclusions can be made concerning the electrolyte selectivity status of the FO-membrane 2c. The dilution ratio in the case of glucose at the second side 2b is expected to be between 1:2 and 1:10, more precisely between 1:4 and 1:7 for a FO-membrane 2c having integrity, and with one of the specified electrolyte solutions at the first side 2a. The dilution ratio in the case of water at the second side 2b is expected to be between 1:0 and 1:1, more precisely between 1:0 and 1:0.5 for a FO-membrane 2c having integrity, and with one of the specified electrolyte solutions at the first side 2a. The dilution of water occurs because water molecules from the pure water at the second side 2b will diffuse to the first side 2a and hence concentrate the pure water or at least reduce the pure water flow.

Based on above, a general lack of integrity of the FO-membrane 2c may be determined. However, to in more detail understand the source of any integrity error, the method may include configuring the hydrostatic pressure difference between the first side 2a and the second side 2b. Thereby, it can be determined if the integrity error is caused by convective error and/or diffusive error of the FO-membrane 2c. Hence, if passing S1 includes to passing the low-electrolyte solution at the first side 2a at a lower hydrostatic pressure P1 than the hydrostatic pressure P2 at the second side 2b, a resulting determined lack of integrity of the FO-membrane 2c is indicative of a solute diffusive error of the FO-membrane 2c. Thus, as the hydrostatic pressure P1 is lower at the first side 2a, convective transport from the first side 2a to the second side 2b is disabled. A positive TMP difference (P2 minus P1) is then established from the second side 2b to the first side 2a. The TMP difference is at least 80 mmHg (1.5 psig), in some embodiments more than 200 or 300 mmHg (3.9 to 5.8 psig). Elevated conductivity of the solution generated at the second side 2b is then a result of diffusive electrolyte transport from the first side 2a to the second side 2b. Thereby, one source of error leading to loss of integrity, hence leakage, is removed. In case of integrity, it is expected that water will diffuse from the second side 2b to the first side 2a, or from the first side 2a to the second side 2b, depending on the osmolarity difference. If there is a solute diffusive error, electrolytes will diffuse from the first side 2a to the second side 2b and thereby increase the conductivity of the solution generated from the second side 2b. Such an evaluation may be referred to as a diffusion test.

However, the integrity error may also include a convective error, hence, a leakage. In order to detect such error, the method comprises, after (i) passing S1 the electrolyte solution at the first side 2a at a hydrostatic pressure that is lower than the hydrostatic pressure at the second side 2b, and measuring the conductivity S3 of the solution generated from the second side 2b, (ii) passing S4 the electrolyte solution at the first side 2a at a hydrostatic pressure that is higher than the hydrostatic pressure at the second side 2b. A low-electrolyte flow is provided as previously explained under step S3 and present at the second side 2b. Hence, the method comprises configuring the hydrostatic pressure P1 at the first side 2a such that it is higher than the hydrostatic pressure at the second side 2b. The configuring is for example performed by measuring the hydrostatic pressure P1 at the first side 2a with the pressure sensor 8 and controlling a speed of the drain pump 4 based on the sensed pressure such that the hydrostatic pressure P1 is equal to a desired higher hydrostatic pressure. For example, the configuring comprises decreasing the speed of the drain pump 4, such that the P1 is higher than P2 (P1 > P2). As explained, the hydrostatic pressure P2 at the second side 2b is typically constant, as the outlet port *Lₒᵤₜ* is open to atmospheric pressure via the diluted electrolyte container 32. Hence, by changing the hydrostatic pressure P1 at the first side 2a, the transmembrane pressure (TMP) between the first side 2a and the second side 2b can be changed to a desired TMP. The method step S4 may therefore comprise altering the pressure compared to method step S1, such that the TMP gradient is inverted. Thereafter, the method comprises measuring conductivity S5 of the solution generated from the second side 2b. The measuring conductivity S5 may include monitoring the conductivity for a time period until the conductivity has stabilized. Thus, the conductivity is measured again, while passing the fluids having a hydrostatic pressure difference different than during the previous conductivity measurement at step S3. If a leak exists, electrolyte solution is now allowed to flow through the leak from the first side 2a to the second side 2b. Thus, as the hydrostatic pressure is lower at the second side 2b than at the first side 2a, convective transport from the first side 2a to the second side 2b is possible if a leak exists. In case of a leak, the electrolyte solution that flows into the second side 2b from the first side 2a will increase the conductivity of the solution generated from the second side 2b, compared to the previously measured conductivity under step S3. Thus, the conductivity change will be greater. Hence, the method may include determining a conductivity change based on the conductivity measurement at step S3, determining a conductivity change based on the conductivity change at step S5, comparing the changes, and in case the conductivity change based on the conductivity at step S5 is greater than the one determined based on the conductivity at step S3, a leak is determined as being a result of convective electrolyte transport from first side 2a to second side 2b. In other words, in some embodiments, the method comprises determining S6 that the lack of integrity of the FO-membrane 2c is due to a leak (convective) in the FO-membrane 2c, upon the measured conductivity indicating a greater conductivity change than the conductivity change detected with the higher hydrostatic pressure at the second side 2b. The conductivity changes are determined from the same reference. The herein tested direction of convective (leak) flow is from first side 2a to second side 2b, which is of most interest from a risk perspective due to the risk of effluent component contamination of the second side 2b where the dialysis concentrate/solution shall flow. This kind of evaluation may be referred to as a convection test.

The result may be communicated to a user via a user interface (not shown and in communication with control unit 50) of the control arrangement 10, and/or an alarm may by initiated if an integrity error was detected. The user may then take appropriate action, such as replacing the FO-device if there was an integrity error.

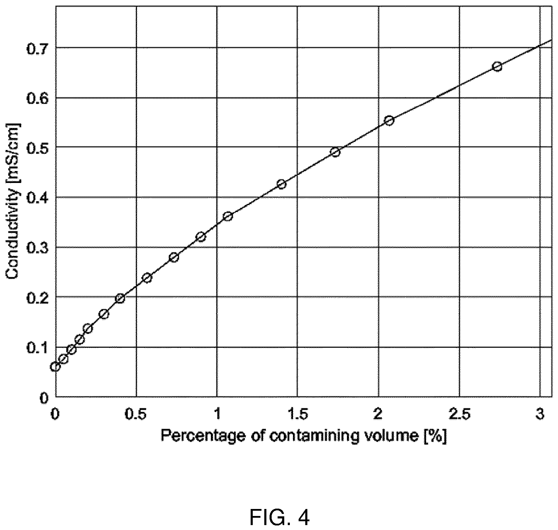

A test was performed to evaluate how the conductivity of a low-electrolyte solution changes with added electrolyte solution. A low electrolyte solution was created by mixing 100ml glucose concentrate and 500ml pure water, representing an anticipated dilution of glucose concentrate to be used in the method. An electrolyte solution was created by mixing 10ml buffer concentrate with 150ml ELIX^{®} water (from Merck), representing a typical dilution of an electrolyte concentrate to be used in the method. The electrolyte solution was stepwisely added in fractions to the low-electrolyte solution volume and mixed, while the conductivity of the mix was measured, to explore the relation between the added fractions of electrolyte solution, and the low-electrolyte solution volume and conductivity. The result is illustrated in Fig. 4, with the conductivity of the mix on one axis in mS/cm and the percentage of electrolyte solution in the total mix on the other axis (percentage of contaminating volume). A baseline conductivity of 0.06 mS/cm originates from the conductivity of the low-electrolyte solution without added electrolyte solution. Such conductivity comes from the low-electrolyte's content of hydrochloric acid. As can be seen from the diagram, a distinct increase of the conductivity was observed with added fractions/volumes of the electrolyte solution.

Tests with the method were also performed using a FO-membrane having integrity (intact membrane) and thereafter with the same membrane having a single fiber ruptured (membrane with single rupture). The FO-membrane used was from Aquaporin^{™}, model HFFO2 (Hollow Fiber Forward Osmosis 2). The result of the tests is illustrated in Table 1.

**Table 1: Resulting conductivity of solution generated at second side during tests.**

| **Kind of membrane** | **Kind of test** | **Qfeed (ml/min)** | **Qdraw (ml/min)** | **TMP (mmHg)** | **Qdraw post (ml/min)** | **Measured conductivity (mS/cm)** |
|---|---|---|---|---|---|---|
| Intact membrane | Diffusion test | 100 | 10 | 82 | 76.2 | 0.57 |
| Intact membrane | Convection test | 100 | 10 | -180 | 80.4 | 0.62 |
| Membrane with single rupture | Diffusion test | 100 | 10 | 90 | 75 | 0.45 |
| Membrane with single rupture | Convection test | 100 | 10 | -180 | 86.6 | 1.45 |

The low-electrolyte solution was a glucose solution having 50% glucose concentrate and 50% water. The electrolyte solution was a buffer solution with electrolyte concentrate diluted with water 1:19. The transmembrane pressure TMP was measured as P2 minus P1. The same FO-membrane was used throughout the tests.

The two first tests in Table 1 were performed with an intact FO-membrane. The conductivity on the generated solution from the second side 2b was elevated compared to the original glucose solution conductivity (0.06 mS/cm), suggesting that electrolytes have been transported from the first side 2a to the second side 2b. The fact that the conductivity elevation is little impacted by the TMP indicates that the conductivity elevation is a result of diffusive transport of electrolytes across the membrane rather than convective.

The two last tests in Table 1 were performed with the same FO-membrane used during the first tests, but with a single fiber ruptured. When TMP had a positive sign (thus disabling convective transport from the first side 2a to the second side 2b), the conductivity of the solution generated at second side 2b was similar (actually somewhat lower) to the ones observed with the intact membrane. As TMP was changed to be negative (thus enabling convective transport from the first side 2a to the second side 2b), the conductivity of the solution generated at the second side 2b became significantly higher than for all other tests indicating that convective transport is occurring. Thus, the ruptured fiber could be detected.

When changing TMP from 82 to -180 mmHg during tests 1 and 2 (intact FO-membrane), the flow rate from *Lₒᵤₜ* increased by 4.2 ml/min due to a resulting increased driving force for diffusive water transport across the membrane. As nearly the same TMP change was introduced during tests 3 and 4 (FO-membrane with fiber rupture), the flow rate from *Lₒᵤₜ* increased by 11.6 ml/min. If it is assumed that the TMP-driven diffusive water transport rate increments are identical between the intact and ruptured membranes, the increment difference (11.6 - 4.4 = 7.2 ml/min) is the convective flow of electrolyte solution to the second side 2b. This means that the generated fluid from the second side 2b contained 8.3% feed solution. The method sensitivity may be increased by using a TMP much lower than - 180 mmHg during the convection test.

The present disclosure also includes a control arrangement 10 for evaluating integrity of a forward osmosis (FO) membrane 2c of a FO-device 2 in a dialysis solution generation apparatus 1. The FO-device 2 is configured to be used in a FO session for diluting a dialysis concentrate in a process of producing a dialysis solution. Hence, the FO session refers to diluting a dialysis concentrate. FO-membrane 2c separates a first side 2a from a second side 2b of the FO-device 2. The control arrangement 10 comprises an effluent pump 3 configured to provide a flow of electrolyte solution. The control arrangement 10 further comprises a concentrate pump 5 configured to provide a flow of low-electrolyte solution. The control arrangement 10 also comprises a conductivity sensor 7 configured to sense a conductivity of a solution generated from the second side 2b. The control arrangement 10 is configured to pass electrolyte solution at the first side 2a, using the effluent pump 3. The control arrangement 10 is also configured to pass low-electrolyte solution at the second side 2b, using the concentrate pump 5. The control arrangement 10 is further configured to measure conductivity, using the conductivity sensor 7, of a solution generated from the second side 2b. The control arrangement 10 is also configured to evaluate the integrity of the FO-membrane based on whether the measured conductivity meets conductivity criteria, wherein the conductivity criteria include or define the conductivity of a solution generated from the second side with an equivalent electrolyte solution and an equivalent low-electrolyte solution and using a FO-membrane that is intact or has integrity.

In some embodiments, the control arrangement 10 is configured to determine a lack of integrity of the FO-membrane 2c, upon determining that the measured conductivity indicates a conductivity change from a conductivity of the low-electrolyte solution that is greater than a threshold conductivity change.

In some embodiments, the control arrangement 10 is configured to pass the low-electrolyte solution at the second side 2b at a hydrostatic pressure that is lower than the hydrostatic pressure at the first side 2a, thereby disabling convective transport from the first side 2a to the second side 2b. A determined lack of integrity of the FO-membrane 2c is then indicative of a solute diffusive error of the FO-membrane 2c.

In some embodiments, the control arrangement 10 is configured (i) to pass the low-electrolyte solution at the second side 2b at a hydrostatic pressure that is higher than the hydrostatic pressure at the first side 2a, (ii) to measure conductivity of the solution generated from the second side, using the conductivity sensor 7, and (iii) to determine that the lack of integrity of the FO-membrane 2c is due to a leak in the FO-membrane 2c upon determining that the measured conductivity is indicates a greater conductivity change than the conductivity change generated at the higher hydrostatic pressure at the second side.

In some embodiments, the control arrangement 10 is configured to pass the low-electrolyte solution at the second side 2b at an osmotic pressure that is higher than the osmotic pressure at the first side 2a, thereby enabling diffusive water transport from the first side 2a to the second side 2b. Or, the control arrangement 10 is configured to pass low-electrolyte solution at the second side at a lower osmotic pressure than the osmotic pressure at the first side 2a, thereby enabling diffusive water transport from the second side 2b to the first side 2a.

In some embodiments, the control arrangement 10 is configured to control the hydrostatic pressure using the drain pump 4. The control arrangement 10 may be configured to perform any one of the herein described aspects, embodiments or examples.

The scope of the present application is delimited by the appended set of claims.

## Claims

1. A method for evaluating the integrity of a forward osmosis (FO) membrane (2c) of a FO-device (2) in a dialysis solution generation apparatus (1), the FO-device (2) configured to be used in a FO session for diluting a dialysis concentrate in a process of producing a dialysis solution, the FO-membrane (2c) separating a first side (2a) and a second side (2b) of a FO-device (2), the method comprising:
passing (S1) electrolyte solution at the first side (2a);
passing (S2) low-electrolyte solution at the second side (2b);
measuring a conductivity (S3) of a solution generated from the second side (2b); and
evaluating the integrity (S6) of the FO-membrane (2c) based on whether the measured conductivity meets conductivity criteria, wherein the conductivity criteria include a conductivity of a solution generated from the second side (2b) with an equivalent electrolyte solution and an equivalent low-electrolyte solution using a FO-membrane having integrity.

2. The method according to claim 1, wherein evaluating the integrity (S6) comprises determining a lack of integrity of the FO-membrane, upon the measured conductivity indicating a conductivity change from a conductivity of the low-electrolyte solution that is greater than a threshold.

3. The method according to claim 2, wherein the threshold is based on a conductivity of the low-electrolyte solution and a predetermined dilution ratio of the low-electrolyte solution in the FO-device (2).

4. The method according to claim 2 or 3, comprising passing (S1) the electrolyte solution at the first side (2a) at a hydrostatic pressure that is lower than a hydrostatic pressure at the second side (2b), thereby disabling convective transport from the first side (2a) to the second side (2b), whereby a determined lack of integrity of the FO-membrane indicates a solute diffusive error of the FO-membrane.

5. The method according to claim 4, comprising passing (S4) the electrolyte solution at the first side (2a) at a hydrostatic pressure that is higher than the hydrostatic pressure at the second side (2b); measuring a conductivity (S5) of the solution generated from the second side (2b); and determining (S6) that the lack of integrity of the FO-membrane is due to a leak in the FO-membrane upon the measured conductivity indicating a greater conductivity change than a conductivity change detected with the lower hydrostatic pressure at the first side (2a).

6. The method according to any one of the preceding claims, comprising (i) passing (S2) low-electrolyte solution at the second side (2b) at an osmotic pressure that is higher than the osmotic pressure at the first side (2a), enabling diffusive water transport from the first side (2a) to the second side (2b), or (ii) passing (S2) low-electrolyte solution at the second side (2b) at an osmotic pressure that is lower than the osmotic pressure at the first side (2a), enabling diffusive water transport from the second side (2b) to the first side (2a).

7. The method according to any one of the preceding claims, wherein the low-electrolyte solution is a glucose solution.

8. The method according to any one of the claims 1 to 6, wherein the low-electrolyte solution is water.

9. The method according to any one of the preceding claims, wherein the electrolyte solution is effluent from a dialysis treatment.

10. The method according to any one of the claims 1 to 8, wherein the electrolyte solution is a diluted electrolyte concentrate.

11. The method according to any one of the preceding claims, wherein the low-electrolyte solution has a conductivity in a range from zero to 0.5 mS/cm, optionally below 0.1 mS/cm.

12. A control arrangement (10) for evaluating the integrity of a forward osmosis (FO) membrane (2c) of a FO-device (2) in a dialysis solution generation apparatus (1), the FO-device (2) configured to be used in a FO session for diluting a dialysis concentrate in a process of producing a dialysis solution, wherein the FO-membrane (2c) separates a first side (2a) and a second side (2b) of the FO-device (2), the control arrangement (10) comprising:
an effluent pump (3) configured to provide a flow of electrolyte solution;
a concentrate pump (5) configured to provide a flow of low-electrolyte solution,
a conductivity sensor (7) configured to sense a conductivity of a solution generated from the second side (2b), and
wherein the control arrangement (10) is configured to:
pass electrolyte solution at the first side (2a), using the effluent pump (3);
pass low-electrolyte solution at the second side (2b), using the concentrate pump (5);
measure a conductivity, using the conductivity sensor (7), of a solution generated from the second side (2b); and
evaluate the integrity of the FO-membrane (2c) based on whether the measured conductivity meets conductivity criteria, wherein the conductivity criteria include a conductivity of a solution generated from the second side (2b) with an equivalent electrolyte solution and an equivalent low-electrolyte solution using a FO-membrane having integrity.

13. A solution generation apparatus (1) for generating dialysis solution, the apparatus comprising a forward osmosis device (2) including a FO-membrane (2c) that separates a first side (2a) and a second side (2b) of the FO-device (2), the apparatus further comprising a control arrangement (10) according to claim 12.

## Patentansprüche

1. Verfahren zum Bewerten der Unversehrtheit einer Vorwärtsosmose (FO, Forward Osmosis) -Membran (2c) einer FO-Vorrichtung (2) in einer Einrichtung (1) zur Erzeugung einer Dialyselösung, wobei die FO-Vorrichtung (2) dafür ausgelegt ist, in einer FO-Sitzung zum Verdünnen eines Dialysekonzentrats in einem Verfahren zum Herstellen einer Dialyselösung verwendet zu werden, wobei die FO-Membran (2c) eine erste Seite (2a) und eine zweite Seite (2b) einer FO-Vorrichtung (2) trennt, wobei das Verfahren umfasst:
Einleiten (S1) von Elektrolytlösung an der ersten Seite (2a);
Einleiten (S2) von elektrolytarmer Lösung an der zweiten Seite (2b);
Messen der Leitfähigkeit (S3) einer erzeugten Lösung von der zweiten Seite (2b); und
Bewerten der Unversehrtheit (S6) der FO-Membran (2c) basierend darauf, ob die gemessene Leitfähigkeit Leitfähigkeitskriterien erfüllt, wobei die Leitfähigkeitskriterien eine Leitfähigkeit einer erzeugten Lösung von der zweiten Seite (2b) beinhalten, mit einer äquivalenten Elektrolytlösung und einer äquivalenten elektrolytarmen Lösung unter Verwendung einer FO-Membran, die Unversehrtheit aufweist.

2. Verfahren nach Anspruch 1, wobei das Bewerten der Unversehrtheit (S6) das Bestimmen eines Mangels an Unversehrtheit der FO-Membran umfasst, wenn die gemessene Leitfähigkeit eine Leitfähigkeitsänderung gegenüber einer Leitfähigkeit der elektrolytarmen Lösung anzeigt, die größer als ein Schwellenwert ist.

3. Verfahren nach Anspruch 2, wobei der Schwellenwert auf einer Leitfähigkeit der elektrolytarmen Lösung und einem vorbestimmten Verdünnungsverhältnis der elektrolytarmen Lösung in der FO-Vorrichtung (2) basiert.

4. Verfahren nach Anspruch 2 oder 3, umfassend das Einleiten (S1) der Elektrolytlösung an der ersten Seite (2a) bei einem hydrostatischen Druck, der niedriger ist als ein hydrostatischer Druck auf der zweiten Seite (2b), wodurch der konvektive Transport von der ersten Seite (2a) zur zweiten Seite (2b) unterbunden wird, wobei ein bestimmter Mangel an Unversehrtheit der FO-Membran einen Lösungsmittel-Diffusionsfehler der FO-Membran anzeigt.

5. Verfahren nach Anspruch 4, umfassend das Einleiten (S4) der Elektrolytlösung an der ersten Seite (2a) bei einem hydrostatischen Druck, der höher ist als der hydrostatische Druck auf der zweiten Seite (2b); das Messen einer Leitfähigkeit (S5) der erzeugten Lösung von der zweiten Seite (2b); und das Bestimmen (S6), dass der Mangel an Unversehrtheit der FO-Membran auf ein Leck in der FO-Membran zurückzuführen ist, wenn die gemessene Leitfähigkeit eine größere Leitfähigkeitsänderung anzeigt als eine Leitfähigkeitsänderung, die mit dem niedrigeren hydrostatischen Druck auf der ersten Seite (2a) erkannt wurde.

6. Verfahren nach einem der vorstehenden Ansprüche, umfassend (i) das Einleiten (S2) einer elektrolytarmen Lösung an der zweiten Seite (2b) bei einem osmotischen Druck, der höher ist als der osmotische Druck auf der ersten Seite (2a), wodurch ein diffusiver Wassertransport von der ersten Seite (2a) zur zweiten Seite (2b) ermöglicht wird, oder (ii) Einleiten (S2) einer elektrolytarmen Lösung an der zweiten Seite (2b) bei einem osmotischen Druck, der niedriger ist als der osmotische Druck auf der ersten Seite (2a), was einen diffusiven Wassertransport von der zweiten Seite (2b) zur ersten Seite (2a) ermöglicht.

7. Verfahren nach einem der vorstehenden Ansprüche, wobei die elektrolytarme Lösung eine Glukoselösung ist.

8. Verfahren nach einem der Ansprüche 1 bis 6, wobei die elektrolytarme Lösung Wasser ist.

9. Verfahren nach einem der vorstehenden Ansprüche, wobei die elektrolytarme Lösung Ausspülwasser aus einer Dialysebehandlung ist.

10. Verfahren nach einem der Ansprüche 1 bis 8, wobei die elektrolytarme Lösung ein verdünntes Elektrolytkonzentrat ist.

11. Verfahren nach einem der vorstehenden Ansprüche, wobei die elektrolytarme Lösung eine Leitfähigkeit in einem Bereich von null bis 0,5 mS/cm, optional unter 0,1 mS/cm, aufweist.

12. Steuerungsanordnung (10) zum Bewerten der Unversehrtheit einer Vorwärtsosmose (FO, Forward Osmosis) -Membran (2c) einer FO-Vorrichtung (2) in einer Einrichtung (1) zur Erzeugung einer Dialyselösung, wobei die FO-Vorrichtung (2) dafür ausgelegt ist, in einer FO-Sitzung zum Verdünnen eines Dialysekonzentrats in einem Verfahren zum Herstellen einer Dialyselösung verwendet zu werden, wobei die FO-Membran (2c) eine erste Seite (2a) und eine zweite Seite (2b) einer FO-Vorrichtung (2) trennt, wobei die Steuerungsanordnung (10) umfasst:
eine Ausspülwasserpumpe (3), die dafür ausgelegt ist, einen Strom von Elektrolytlösung zu liefern;
eine Konzentratpumpe (5), die dafür ausgelegt ist, einen Strom von elektrolytarmer Lösung zu liefern,
einen Leitfähigkeitssensor (7), der dafür ausgelegt ist, eine Leitfähigkeit einer erzeugten Lösung von der zweiten Seite (2b) zu erfassen, und
wobei die Steuerungsanordnung (10) ausgelegt ist zum:
Einleiten von Elektrolytlösung an der ersten Seite (2a) mit Hilfe der Ausspülwasserpumpe (3);
Einleiten von elektrolytarmer Lösung an der zweiten Seite (2b) mit Hilfe der Konzentratpumpe (5);
Messen einer Leitfähigkeit, mithilfe des Leitfähigkeitssensors (7), einer erzeugten Lösung von der zweiten Seite (2b); und
Bewerten der Unversehrtheit der FO-Membran (2c) basierend darauf, ob die gemessene Leitfähigkeit Leitfähigkeitskriterien erfüllt, wobei die Leitfähigkeitskriterien eine Leitfähigkeit einer erzeugten Lösung von der zweiten Seite (2b) beinhalten, mit einer äquivalenten Elektrolytlösung und einer äquivalenten elektrolytarmen Lösung unter Verwendung einer FO-Membran, die Unversehrtheit aufweist.

13. Lösungserzeugungseinrichtung (1) zum Erzeugen einer Dialyselösung, wobei die Einrichtung eine Vorwärtsosmosevorrichtung (2) mit einer FO-Membran (2c), die eine erste Seite (2a) und eine zweite Seite (2b) der FO-Vorrichtung (2) trennt, umfasst, wobei die Einrichtung ferner eine Steuerungsanordnung (10) nach Anspruch 12 umfasst.

## Revendications

1. Procédé d'évaluation de l'intégrité d'une membrane (2c) d'osmose directe (FO) d'un dispositif FO (2) dans un appareil de génération de solution de dialyse (1), le dispositif FO (2) étant configuré pour être utilisé dans une session FO pour diluer un concentré de dialyse dans un processus de production d'une solution de dialyse, la membrane FO (2c) séparant un premier côté (2a) et un second côté (2b) d'un dispositif FO (2), le procédé comprenant :
le passage (S1) d'une solution électrolytique au niveau du premier côté (2a) ;
le passage (S2) d'une solution faiblement électrolytique au niveau du second côté (2b) ;
la mesure de la conductivité (S3) d'une solution générée depuis le second côté (2b) ; et
l'évaluation de l'intégrité (S6) de la membrane FO (2c) selon que la conductivité mesurée répond ou non à des critères de conductivité, dans lequel les critères de conductivité comprennent une conductivité d'une solution générée depuis le second côté (2b) avec une solution électrolytique équivalente et une solution faiblement électrolytique équivalente à l'aide d'une membrane FO ayant une intégrité.

2. Procédé selon la revendication 1, dans lequel l'évaluation de l'intégrité (S6) comprend la détermination d'un manque d'intégrité de la membrane FO, lorsque la conductivité mesurée indique un changement de conductivité par rapport à une conductivité de la solution faiblement électrolytique qui est supérieure à un seuil.

3. Procédé selon la revendication 2, dans lequel le seuil est basé sur une conductivité de la solution faiblement électrolytique et un rapport de dilution prédéterminé de la solution faiblement électrolytique dans le dispositif FO (2).

4. Procédé selon la revendication 2 ou 3, comprenant le passage (S1) de la solution électrolytique au niveau du premier côté (2a) à une pression hydrostatique qui est inférieure à une pression hydrostatique au niveau du second côté (2b), désactivant ainsi un transport convectif du premier côté (2a) au second côté (2b), moyennant quoi un manque déterminé d'intégrité de la membrane FO indique une erreur de diffusion de soluté de la membrane FO.

5. Procédé selon la revendication 4, comprenant le passage (S4) de la solution électrolytique au niveau du premier côté (2a) à une pression hydrostatique qui est supérieure à la pression hydrostatique au niveau du second côté (2b) ; la mesure d'une conductivité (S5) de la solution générée depuis le second côté (2b) ; et la détermination (S6) que le manque d'intégrité de la membrane FO est dû à une fuite dans la membrane FO lorsque la conductivité mesurée indique un changement de conductivité plus important qu'un changement de conductivité détecté avec la pression hydrostatique plus faible au niveau du premier côté (2a).

6. Procédé selon l'une quelconque des revendications précédentes, comprenant (i) le passage (S2) de la solution faiblement électrolytique au niveau du second côté (2b) à une pression osmotique qui est supérieure à la pression osmotique au niveau du premier côté (2a), permettant un transport diffusif de l'eau du premier côté (2a) au second côté (2b), ou (ii) le passage (S2) d'une solution faiblement électrolytique au niveau du second côté (2b) à une pression osmotique qui est inférieure à la pression osmotique au niveau du premier côté (2a), permettant un transport diffusif de l'eau du second côté (2b) au premier côté (2a).

7. Procédé selon l'une quelconque des revendications précédentes, dans lequel la solution faiblement électrolytique est une solution de glucose.

8. Procédé selon l'une quelconque des revendications 1 à 6, dans lequel la solution faiblement électrolytique est l'eau.

9. Procédé selon l'une quelconque des revendications précédentes, dans lequel la solution électrolytique est un effluent d'un traitement par dialyse.

10. Procédé selon l'une quelconque des revendications 1 à 8, dans lequel la solution électrolytique est un concentré électrolytique dilué.

11. Procédé selon l'une quelconque des revendications précédentes, dans lequel la solution faiblement électrolytique a une conductivité dans la plage de zéro à 0,5 mS/cm, éventuellement inférieure à 0,1 mS/cm.

12. Agencement de commande (10) permettant d'évaluer l'intégrité d'une membrane (2c) d'osmose directe (FO) d'un dispositif FO (2) dans un appareil de génération de solution de dialyse (1), le dispositif FO (2) étant configuré pour être utilisé dans une session FO pour diluer un concentré de dialyse dans un processus de production d'une solution de dialyse, dans lequel la membrane FO (2c) sépare un premier côté (2a) et un second côté (2b) du dispositif FO (2), l'agencement de commande (10) comprenant :
une pompe d'effluent (3) configurée pour fournir un flux de solution électrolytique ;
une pompe de concentré (5) configurée pour fournir un flux de solution faiblement électrolytique,
un capteur de conductivité (7) configuré pour détecter la conductivité d'une solution générée depuis le second côté (2b), et
dans lequel l'agencement de commande (10) est configuré pour :
faire passer la solution électrolytique au niveau du premier côté (2a), à l'aide de la pompe d'effluent (3) ;
faire passer la solution faiblement électrolytique au niveau du second côté (2b), à l'aide de la pompe de concentré (5) ;
mesure la conductivité, à l'aide du capteur de conductivité (7), d'une solution générée depuis le second côté (2b) ; et
évaluer l'intégrité de la membrane FO (2c) en fonction de si la conductivité mesurée répond à des critères de conductivité, dans lequel les critères de conductivité comprennent une conductivité d'une solution générée depuis le second côté (2b) avec une solution électrolytique équivalente et une solution faiblement électrolytique équivalente à l'aide d'une membrane FO ayant une intégrité.

13. Appareil de génération de solution (1) permettant de générer une solution de dialyse, l'appareil comprenant un dispositif d'osmose directe (2) comprenant une membrane FO (2c) qui sépare un premier côté (2a) et un second côté (2b) du dispositif FO (2), l'appareil comprenant en outre un agencement de commande (10) selon la revendication 12.
